# EUROPEAN PATENT APPLICATION

(11) **EP 2 216 019 A2**
(43) Date of publication of application: **11.08.2010**
(21) Application number: 10162594.5
(22) Date of filing: 02.03.2006
(51) Int. Cl.: A61K 31/13, A61P 27/02

(54) **Ophthalmic uses of S1P receptor modulators**

(30) Priority: 04.03.2005 GB 0504544
(62) Divisional of application: 06723169.6
(71) Applicant: Novartis AG, 4056 Basel (CH); Novartis Pharma GmbH, 1230 Wien (AT)
(72) Inventor: Lambrou, George N., 10676, Athens (GR); Latour, Elisabeth Jeanne, 75014, Paris (FR)
(74) Representative: Rouquayrol, Céline Hélène

(57) **Abstract**

The present invention pertains to the use of a S1 P receptor agonist in the manufacture of a medicament in the treatment of an ocular disorder.

## Description

The present invention relates to the use of a an S1P receptor agonist in the manufacture of a medicament for the treatment of ocular disorders.

Ocular disorders which may be treated according to this invention include typically an ocular disease and disorder which may directly or indirectly involve the degeneration of retinal or corneal cells, in particular by apoptosis. Ocular disorders, as used herein, include ischemic retinopathies in general, anterior ischemic optic neuropathy, all forms of optic neuritis, age-related macular degeneration (AMD), in its dry forms (dry AMD) and wet forms (wet AMD), diabetic retinopathy, diabetic macular edema (DME), proliferative diabetic retinopathy (PDR), cystoid macular edema (CME), retinal detachment, retinitis pigmentosa (RP), Stargardt's disease, Best's vitelliform retinal degeneration, Leber's congenital amaurosis and other hereditary retinal degenerations, pathologic myopia, retinopathy of prematurity,and Lebers hereditary optic neuropathy, the after effects of corneal transplantation or of refractive corneal surgery, keratoconjunctivitis sicca (KCS) or dry eye and herpes keratitis.

Preferably, said ocular disorders are selected from:
Dry AMD, wet AMD, diabetic retinopathy, diabetic macular edema (DME), proliferative diabetic retinopathy (PDR), retinitis pigmentosa (RP), and keratoconjunctivits sicca (KCS), and even more preferably, said ocular disorders are selected from:
   Dry AMD, wet AMD, DME and PDR.

Also preferably said ocular disorder is PDR.

Also preferably said ocular disorder is DME.

Also preferably said ocular disorder is keratoconjunctivits sicca (KCS).

Highly preferably, said ocular disorders are selected from dry AMD and wet AMD.

In the present description the terms "treatment" or "treat" refer to both prophylactic or preventive treatment as well as curative or disease-modifying treatment, including treatment of patients at risk of contracting the disease or suspected to have contracted the disease as well as patients who are ill or have been diagnosed as suffering from a disease or medical condition.

S1P receptor agonists are compounds which signal as agonists at one or more sphingosine-1 phosphate receptors, e.g. S1P1 to S1P8. Agonist binding to a 51P receptor may e.g. result in dissociation of intracellular heterotrimeric G-proteins into Gα-GTP and Gβy-GTP, and/or increased phosphorylation of the agonist-occupied receptor and activation of downstream signaling pathways/kinases.

S1 P receptor agonists are typically sphingosine analogues, such as 2-substituted 2-aminopropane-1,3-diol or 2-amino-propanol derivatives, e. g. a compound comprising a group of formula X 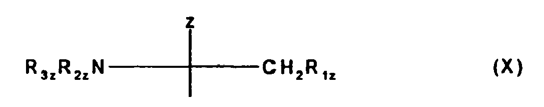 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃₋₈ cycloalkyl, phenyl and phenyl substituted by OH, or CH₂-R_{4z} wherein R_{4z} is OH, acyloxy or a residue of formula (a) 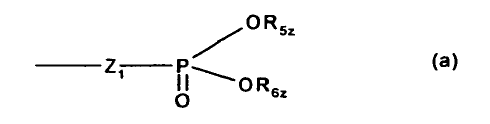 wherein Z₁ is a direct bond or O, preferably O;
each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

Examples of preferred S1 P receptor agonists are, for example:
- Compounds as disclosed in EP627406A1, e.g. a compound of formula I 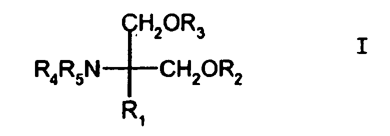 wherein R₁ is a straight- or branched (C₁₂₋₂₂)chain
- which may have in the chain a bond or a hetero atom selected from a double bond, a triple bond, O, S, NR₆. wherein R₆ is H, alkyl, aralkyl, acyl or alkoxycarbonyl, and carbonyl, and/or
- which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenylalkoxy, halophenylalkoxy, phenylalkoxyalkyl, phenoxyalkoxy or phenoxyalkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
and wherein
the alkyl moiety may have
- in the carbon chain, a bond or a heteroatom selected from a double bond, a triple bond, O, S, sulfinyl, sulfonyl, or NR₆, wherein R₆ is as defined above, and
- as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
each of R₂, R₃, R₄ and R₅, independently, is H, C₁₋₄ alkyl or acyl
or a pharmacologically acceptable salt, solvate or hydrate thereof;
- Compounds as disclosed in EP 1002792A1, e.g. a compound of formula II 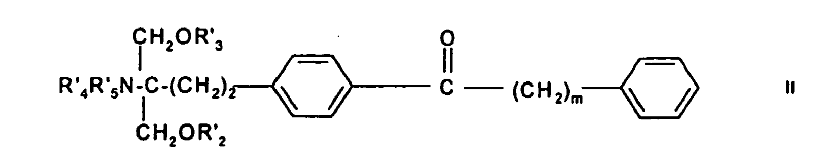
wherein m is 1 to 9 and each of R'₂, R'₃, R'₄ and R'₅, independently, is H, alkyl or acyl, or a pharmacologically acceptable salt, solvate or hydrate thereof;
- Compounds as disclosed in EP0778263 A1, e.g. a compound of formula III 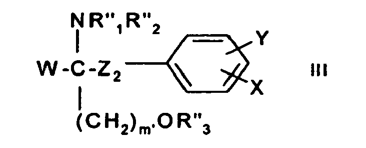
wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH; X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆ alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
each of p and q, independently, is an integer of 1 to 20, with the proviso of 6≤p+q≤23, m' is 1,2 or 3, n is 2 or 3,
each of R"₁, R"₂, R"₃ and R"₄, independently, is H, C₁₋₄alkyl or acyl,
or a pharmacologically acceptable salt, solvate or hydrate thereof,
- Compounds as disclosed in WO02/18395, e.g. a compound of formula IVa or IVb 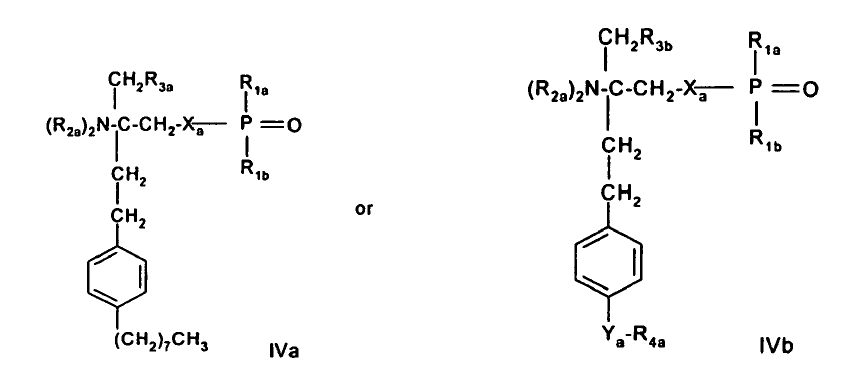
wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄. ₁₄)alkyl or (C₄₋₁₄)alkenyl;
or a pharmacologically acceptable salt, solvate or hydrate thereof;
- Compounds as disclosed in WO 02/076995, e.g. a compound of formula V 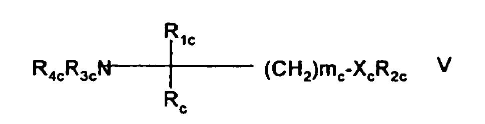
wherein
- m_{c}: is 1, 2 or 3;
- X_{c}: is O or a direct bond;
- R_{1c}: is H; C₁₋₆ alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
- R_{2c}: is 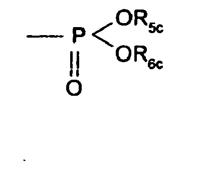 wherein R_{5c} is H or C₁₋₄alkyl optionally substituted by 1, 2 or 3 halogen atoms, and R_{6c} is H or C₁₋₄alkyl optionally substituted by halogen;
each of R_{3c} and R_{4c}, independently, is H, C₁₋₄alkyl optionally substituted by halogen, or acyl, and
- R_{c}: is C₁₃₋₂₀alkyl which may optionally have in the chain an oxygen atom and which may optionally be substituted by nitro, halogen, amino, hydroxy or carboxy; or a residue of formula (a) 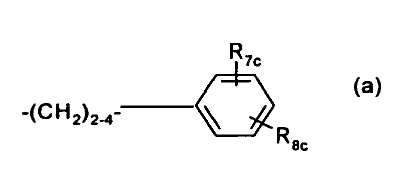
wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,
- R_{c}: being also a residue of formula (a) wherein R_{8c} is C₁₋₄alkoxy when R_{1c} is C₁₋₄alkyl, C₂₋₆alkenyl or C₂₋₆alkynyl,
- or a: compound of formula VI 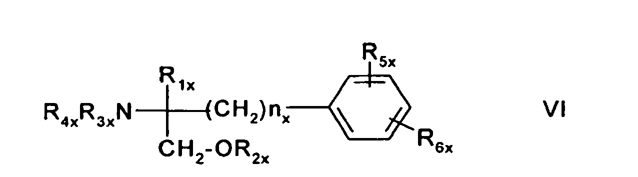
wherein
- nₓ: is 2, 3 or 4
- R₁ₓ: is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
- R₂ₓ: is H, C₁₋₄ alkyl or acyl
each of R₃ₓ and R₄ₓ, independently is H, C₁₋₄alkyl optionally substituted by halogen or acyl,
- R₅ₓ: is H, C₁₋₄alkyl or C₁₋₄alkoxy, and

- R₆ₓ: is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
- R₆ₓ: being also C₄₋₁₄alkoxy when R₁ₓ is C₂₋₄alkyl substituted by OH, or pentyloxy or hexyloxy when R₁ₓ is C₁₋₄akyl,
provided that R₅ₓ is other than phenyl-butylenoxy when either R₅ₓ is H or R₁ₓ is methyl, or a pharmacologically acceptable salt, solvate or hydrate thereof;
- Compounds as disclosed in WO02/06268Al, e.g. a compound of formula VII 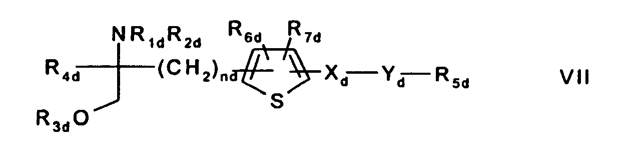 wherein each of R_{1d} and R_{2d}, independently, is H or an amino-protecting group; R_{3d} is hydrogen, a hydroxy-protecting group or a residue of formula 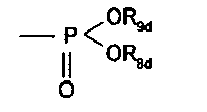 R_{4d} is lower alkyl;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, cycloalkyl, aryl, heterocycle, cycloalkyl substituted by up to three substituents selected from groups a and b, aryl substituted by up to three substituents selected from groups a and b, or heterocycle substituted by up to three substituents selected from groups a and b;
   each of R_{6d} and R_{7d}, independently, is H or a substituent selected from group a;
   each of R_{8d} and R_{9d}, independently, is H or C₁₋₄alkyl optionally substituted by halogen;
   <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano or nitro; and
   <group b > is cycloalkyl, aryl, heterocycle, each being optionally substituted by up to three substituents selected from group a;
   with the proviso that when R_{5d} is hydrogen, Y_{d} is a either a single bond or linear C₁₋₁₀ alkylene, or a pharmacologically acceptable salt or ester thereof;
- Compounds as disclosed in JP-14316985 (JP2002316985), e.g. a compound of formula VIII 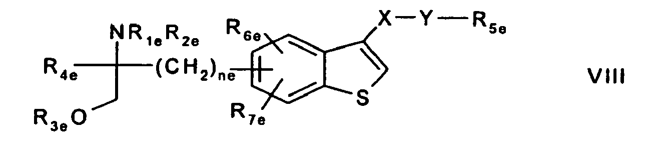 wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt, solvate or hydrate or ester thereof;
- Compounds as disclosed in WO 03/29184 and WO 03/29205, e.g. compounds of formula IX 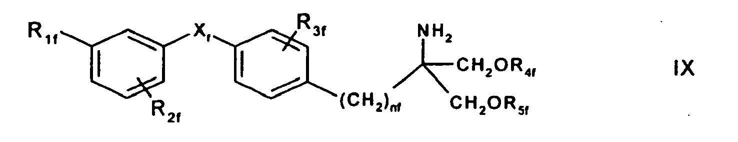 wherein X_{f} is O or S, and R_{1f}, R_{2f}, R_{3f} and n_{f} are as disclosed in WO 03/29184 and WO 03/29205, each of R_{4f} and R_{5f}, independently is H or a residue of formula 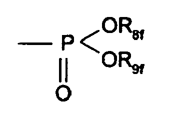 wherein each of R_{8f} and R_{9f}, independently, is H or C₁₋₄alkyl optionally substituted by halogen; e.g. 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol, or a pharmacologically acceptable salt, solvate or hydrate thereof;
- Compounds as disclosed in WO03/062252A1, e.g. a compound of formula X' 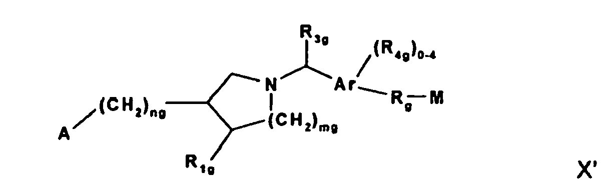 wherein
   Ar is phenyl or naphthyl; each of mg and ng independently is 0 or 1; A is selected from COOH, PO₃H₂, PO₂H SO₃H PO(C₁₋₃alkyl)OH and 1*H*-tetrazol-5-yl; each of R_{1g} and R₂₉ independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R_{g} and M has one of the significances as indicated for B and C, respectively, in WO03/062252A1; or a pharmacologically acceptable salt, solvate or hydrate thereof;
- Compounds as disclosed in WO 03/062248A2, e.g. a compound of formula XI 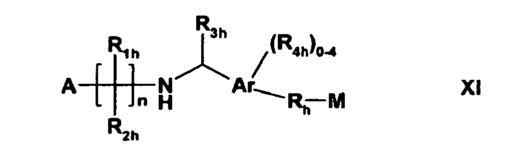 wherein Ar is phenyl or naphthyl; n is 2,3 or 4; A is COOH, 1*H*-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁₋₃alkoxy and -CH(OH)-phenyl wherein said phenyl or phenyl moiety is opitonally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/OH; each R₄ₕ independently is halogeno, OH, COOH, C₁₋₄alkyl, S(O)_{0,1 or2}C₁₋₃alkyl, C₁₋₃alkoxy, C₃₋₆cycloalkoxy, aryl or aralkoxy, wherein the alkyl portions may optionally be substituted by 1-3 halogens; and each of Rg and M has one of the significances as indicated for B and C, respectively, in WO03/062248A2;
- Compounds as disclosed in WO 04/026817A, e.g. compounds of formula XII 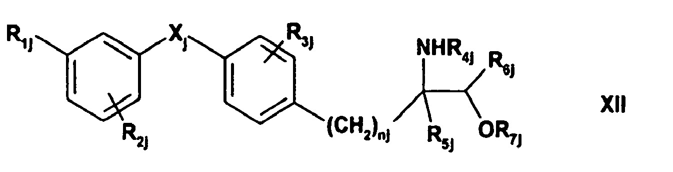 wherein
   R₁ⱼ is halogen, trihalomethyl, C₁₋₄alkyl, C₁₋₄alkoxy, C₁₋₄alkylthio, C₁₋₄alkylsulifinyl, C₁₋₄alkylsulfonyl, aralkyl, optionally substituted phenoxy or aralkyloxy, R₂ⱼ is H, halogen, trihalomethyl, C₁₋₄alkyl, C₁₋₄alkoxy, aralkyl or aralkyloxy, R₃ⱼ is H, halogen, CF₃, C₁₋₄alkyl, C₁₋₄alkoxy, C₁₋₄alkylthio or benzyloxy, R₄ⱼ is H, C₁₋₄alkyl, phenyl, optionally substituted benzyl or benzoyl, or lower aliphatic C ₁₋₅acyl, R₅ⱼ is H, monohalomethyl, C₁₋₄alkyl, C₁₋₄alkoxymethyl, C₁₋₄alkylthiomethyl, hydroxyethyl, hydroxypropyl, phenyl, aralkyl, C₂₋₄alkenyl or -alkynyl, each of R₆ⱼ and R₇ⱼ, independently, is H or C₁₋₄alkyl, or R₇ⱼ being also a residue of formula 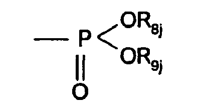 wherein each of R₈ⱼ and R₉ⱼ, independently, is H or C₁₋₄alkyl optionally substituted by halogen Xⱼ is O, S, SO or SO₂ and nⱼ is an integer of 1 to 4, e.g. 2-amino-4-[4-(3-benzyloxyphenylthio)-2-chlorophenyl]-2-methylbutane-1-ol or 2-amino-4-[4-(3-benzyloxyphenylthio)-2-chlorophenyl]-2-ethylbutane-1-ol;
- Compounds as disclosed in WO 04/103306A, WO 05/000833, WO 05/103309 or WO 05/113330, e.g. compounds of formula XIIIa or Xlllb 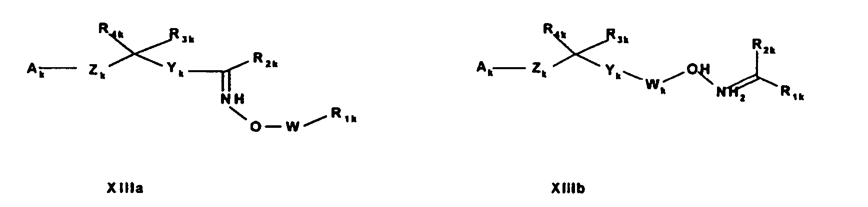 wherein
   Aₖ is COOR₅ₖ, OPO(OR₅ₖ)₂, PO(OR₅ₖ)₂, SO₂OR₅ₖ, POR₅ₖOR₅ₖ or 1*H*-tetrazol-5-yl, R₅ₖ being H or C₁₋₆alkyl;
   Wₖ is a bond, C₁₋₃alkylene or C₂₋₃alkenylene;
   Yₖ is C₆₋₁₀aryl or C₃₋₉heteroaryl, optionally substituted by 1 to 3 radicals selected from halogene, OH, NO₂, C₁₋₆alkyl, C₁₋₆alkoxy; halo-substituted C₁₋₆alkyl and halo-substituted C₁₋₆alkoxy;
   Zₖ is a heterocyclic group as indicated in WO 04/103306A, e.g. azetidine;
   R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉eheteroaryl, C₃₋₉heteroarylC₁₋₄alkyl, C₃₋₈cycloalkyl, C₃₋₈cycloalkylC₁₋₄alkyl, C₃₋₈heterocycloalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋₆alkyl or -C₁₋₆alkoxy;
   R₂ₖ is H, C₁₋₆alkyl, halo substituted C₁₋₆alkyl, C₂₋₆alkenyl or C₂₋₆alkynyl: and
   each of Pₐₖ or R₄ₖ, independently, is H, halogen, OH, C₁₋₆alkyl, C₁₋₆alkoxy or halo substituted C₁₋₆alkyl or C₁₋₆alkoxy;
   and the N-oxide derivatives thereof or prodrugs thereof,
   or a pharmacologically acceptable salt, solvate or hydrate thereof.

According to a further embodiment of the invention, a S1P receptor agonist for use in the invention may also be a selective S1P1 receptor, e.g. a compound which possesses a selectivity for the S1P1 receptor over the S1P3 receptor of at least 20 fold, e.g. 100, 500, 1000 or 2000 fold, as measured by the ratio of EC₅₀ for the S1P1 receptor to the EC₅₀ for the S1P3 receptor as evaluated in a ³⁵S-GTPγS binding assay, said compound having an EC₅₀ for binding to the S1P1 receptor of 100 nM or less as evaluated by the ³⁵S-GTPγS binding assay. Representative S1P1 receptor agonists are e.g. the compounds listed in WO 03/061567, the contents of which being incorporated herein by reference, for instance a compound of formula XIV or XV

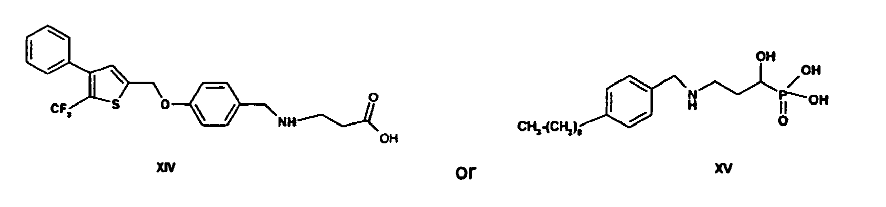

When the compounds of formulae I to XV have one or more asymmetric centers in the molecule, the present invention is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of above formulae may exist in free or salt form. Examples of pharmaceutically acceptable salts of the compounds of the above formulae include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the present invention encompass hydrate and solvate forms.

Acyl as indicated above may be a residue Rγ-CO- wherein Ry is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), e.g. the hydrochloride, as shown:

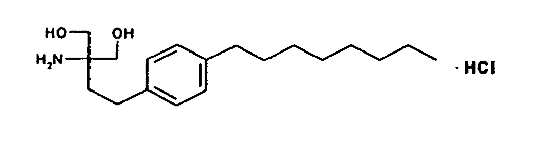

A preferred compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g. the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula IVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula V is phosphoric acid mono-[(R)-2-amino-2-methyl-4-(4-pentyloxy-phenyl)-butyl]ester.

A preferred compound of formula VIII is (2R)-2-amino-4-[3-(4-cyclohexyloxybutyl)-benzo[b]thien-6-yl]-2-methylbutan-1-ol.

A preferred compound of formula IX is a compound wherein X_{f} is S or O, R_{1f} is benzyloxy, R_{2f}, R_{4f} and R_{5fj} are each H, R_{3f} is Cl and n_{f} is 2.

A preferred compound of formula XII is a compound wherein Xⱼ is S or O, R₁ⱼ is benzyloxy, R₂ⱼ, R₄ⱼ, R₆ⱼ and R₇ⱼ are each H, R₃ⱼ is Cl, R₅ⱼ is hydroxyethyl or hydroxypropyl and nⱼ is 2.

Binding affinity of S1P receptor agonists to individual human S1P receptors may be determined in following assays:

### Transient transfection of human S1P receptors into HEK293 cells

EDG receptors and G₁ proteins are cloned, and equal amounts of 4 cDNAs for the EDG receptor, Gᵢ-α, G_{i-β} and G_{i-γ} are mixed and used to transfect monolayers of HEK293 cells using the calcium phosphate precipitate method (M. Wigler et al., Cell. 1977;11;223 and DS. lm et al., Mol. Pharmacol. 2000;57;753). Briefly, a DNA mixture containing 25 µg of DNA and 0.25 M CaCl is added to HEPES-buffered 2 mM Na₂HPO₄. Subconfluent monolayers of HEK293 cells are poisoned with 25 mM chloroquine, and the DNA precipitate is then applied to the cells. After 4 h, the monolayers are washed with phosphate-buffered saline and refed media (90% 1:1 Dulbecco's modified essential media (DMEM):Fa12 + 10% fetal bovine serum). The cells are harvested 48-72 h after addition of the DNA by scraping in HME buffer (in mM: 20 HEPES, 5 MgCl₂, 1 EDTA, pH 7.4) containing 10% sucrose on ice, and disrupted using a Dounce homogenizer. After centrifugation at 800×g, the supernatant is diluted with HME without sucrose and centrifuged at 100,000×g for 1 h. The resulting pellet is rehomogenized and centrifuged a second hour at 100,000×g. This crude membrane pellet is resuspended in HME with sucrose, aliquoted, and snap-frozen by immersion in liquid nitrogen. The membranes are stored at 70°C. Protein concentration is determined spectroscopically by Bradford protein assay.

### GTPγS binding assay using S1P receptor/HEK293 membrane preparations

GTPγS binding experiments are performed as described by DS. lm et al., Mol. Pharmacol. 2000; 57:753. Ligand-mediated GTPγS binding to G-proteins is measured in GTP binding buffer (in mM: 50 HEPES, 100 NaCl, 10 MgCl₂, pH 7.5) using 25 µg of a membrane preparation from transiently transfected HEK293 cells. Ligand is added to membranes in the presence of 10 µM GDP and 0.1 nM [³⁵S]GTPγS (1200 Ci/mmol) and incubated at 30°C for 30 min. Bound GTPγS is separated from unbound using the Brandel harvester (Gaithersburg, MD) and counted with a liquid scintillation counter.

Compounds of formula A are disclosed e.g. in WO 94/09010, WO 95/16691, WO 96/41807, USP 5,362,718 or WO 99/15530 which are incorporated herein by reference. They may be prepared as disclosed or by analogy to the procedures described in these references.

In a series of further specific or alternative embodiments, the present invention also provides:
1.1. A method for treating an ocular disorder, said method comprising administering to an affected individual a therapeutically effective amount of a S1 P receptor agonist.
   Preferred S1P receptor agonist is Compound A, B or C, (2R)-2-amino-4-[3-(4-cyclohexyloxybutyl)-benzo[b]thien-6-yl]-2-methylbutan-1-ol, or a compound of formula IX wherein X_{f} is S or O, R_{1f} is benzyloxy, R_{2f}, R_{4f} and R_{5fj} are each H, R_{3f} is Cl and n_{f} is 2.

As used herein, administration is preferably pertaining to oral, rectal, parenteral and topical administration. An even more preferred administration pertains to topical administration.

Efficacy in the described ocular disorders might be established for example in the following animal models:
1) Genetic animal models for retinal degeneration, e.g. rd mouse (as described in Li et al., Invest. Ophthalmol. Vis. Sci. 2001 ; 42: 2981-2989), Rpe65-deficient mouse (Van Hooser et al., PNAS 2000. ; 97: 8623-8628), RCS rat (Faktorovich et al., Nature 1990; 347:83-86), rds mouse (Ali et al., Nature Genetics 2000, 25 : 306-310), rcd1 dog (Suber et al., PNAS 1993; 90: 3968-3972)
2) Experimental retinal degeneration induced by
   - light exposure in mice (as described in Wenzel et al., Invest. Ophthalmol. Vis. Sci. 2001; 42: 1653-1659) or rats (Faktorovich et al., J. Neurosci: 1992; 12: 3554-3567)
   - administration of N-methyl-N-nitrosourea (Kiuchi et al., Exp. Eye Res. 2002; 74: 383-392) or sodium iodate (Sorsby & Harding, Vision Res. 1962; 2: 139-148).
3) Experimental model for the injury of the optic nerve (ON)
   - by ON crush in mice (Levkovitch-Verbin et al., Invest. Ophthalmol. Vis. Sci. 2000; 41: 4169-4174) and rats (Yoles and Schwartz, Exp. Neurol. 1998; 153:1-7)
   - by ON transection in rats (as described in Martin et al., Invest. Ophthalmol. Vis. Sci. 2002; 43: 2236-2243, Solomon et al. J. Neurosci. Methods 1996; 70:21-25)
   - by experimental transient (acute) retinal ischemia in rats after ophthalmic vessel ligature (as described in Lafuente et al., Invest. Ophthalmol. Vis. Sci. 2001; 42:2074-2084) or cannulation of the anterior chamber (Buchi et al., Ophthalmologica 1991; 203:138-147)
   - by intraocular endothelin-1 injection in rats (Stokely at al., Invest. Ophthalmol. Vis. Sci. 2002; 43: 3223-3230) or rabbits (Takei et al., Graefes Arch. Clin. Exp. Ophthalmol 1993; 231:476-481)

The pharmaceutical compositions of this invention comprise, for example, enteral or parenteral administration forms from approximately 5 % to approximately 90 %, preferably from approximately 10 % to approximately 80 %, active ingredient. Pharmaceutical compositions according to the invention for enteral or parenteral administration are, for example, in unit dose form, such as in the form of dragées, tablets, capsules or suppositories, and also ampoules. They are prepared in a manner known *per se*, for example by means of conventional mixing, granulating, confectioning, dissolving or lyophilising processes. For example, pharmaceutical compositions for oral administration can be obtained by combining the active ingredient with solid carriers, if desired granulating a resulting mixture, and processing the mixture or granules, if desired or necessary, after the addition of appropriate excipients, into tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, and also binders, such as starch pastes using, for example, com, wheat, rice or potato starch, gelatin, tragacanth, methylcellulose and/or polyvinylpyrrolidone, if desired disintegrators, such as the above-mentioned starches, also carboxymethyl starch, crosslinked polyvinylpyrrolidone, agar, alginic acid or a salt thereof, such as sodium alginate. Excipients are especially flow agents, flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, *inter alia*, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or dragée coatings, for example for identification purposes or to indicate different doses of active ingredient.

Other orally administrable pharmaceutical compositions are hard gelatin capsules and also soft, sealed capsules made of gelatin and a plasticiser, such as glycerol or sorbitol. The hard gelatin capsules may comprise the active ingredient in the form of granules, for example in admixture with fillers, such as lactose, binders, such as starches, and/or glidants, such as talc or magnesium stearate, and if desired with stabilisers. In soft capsules the active ingredient is preferably dissolved or suspended in suitable liquids, such as fatty oils, paraffin oil or liquid polyethylene glycols, it likewise being possible for stabilisers to be added.

Suitable rectally administrable pharmaceutical compositions are, for example, suppositories that consist of a combination of the active ingredient with a suppository base material. Suitable suppository base materials are, for example, natural or synthetic triglycerides, paraffin hydrocarbons, polyethylene glycols or higher alkanols. Gelatin rectal capsules that comprise a combination of the active ingredient with a base material may also be used. Suitable base materials include, for example, liquid triglycerides, polyethylene glycols and paraffin hydrocarbons.

There are suitable for parenteral administration by infusion and/or injection especially aqueous solutions of an active ingredient in water-soluble form, for example in the form of a water-soluble salt, and also suspensions of the active ingredient, such as corresponding oily suspensions, there being used suitable lipophilic solvents or vehicles, such as fatty oils, for example sesame oil, or synthetic fatty acid esters, for example ethyl oleate or triglycerides, or aqueous suspensions that comprise viscosity-increasing substances, for example sodium carboxymethylcellulose, sorbitol and/or dextran, and optionally also stabilisers.

The compounds may also be administered topically in or around the eye, for example as eyedrops, ophthalmic suspensions or ointments, subconjunctival, peribulbar, retrobulbar or intravitreal injections, possibly with the use of slow-release devices, such as conjunctival inserts, microspheres or other periocular or intraocular depot devices.

The dosage of the active ingredient depends on the species of warm-blooded animal, the age and the individual condition and also on the mode of administration. Normally the estimated approximate daily dose in the case of oral administration to a patient weighing approximately 75 kg is from approximately 10 mg to approximately 500 mg.

In the case of topical administration, the approximate estimated daily dosage may vary from 0.001 to 10 mg, depending on the mode of administration. The amount of active ingredient in a topical formulation is typically much lower than in oral or parenteral formulations. Typically the active in a topical formulation would range from 0.01% - 10% by weight of total weight.

## Claims

1. Use of an S1P receptor agonist in the manufacture of a medicament for the treatment of an ocular disorder, wherein the S1P receptor agonist
- is or comprises a group of formula (I): 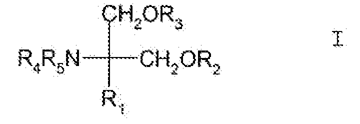 wherein R₁ is a straight- or branched (C₁₂₋₂₂)chain
which may have in the chain a bond or a hetero atom selected from a double bond, a triple bond, O, S, NR₆, wherein R₆ is H, alkyl, aralkyl, acyl or alkoxycarbonyl, and carbonyl, and/or
which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
R₁ is
a phenylalkyl wherein alkyl is a straight- or branched (C₆₋₂₀)carbon chain; or a phenylalkyl wherein alkyl is a straight- or branched (C₁₋₃₀)carbon chain wherein said phenylalkyl is substituted by
a straight- or branched (G₆₋₂₀)carbonchain optionally substituted by halogen, a straight- or branched (C₆₋₂₀)alkoxy chain optionally substitued by halogen,
a straight- or branched (C₆₋₂₀)alkenyloxy,
phenylalkoxy, halophenylalkoxy, phenylalkoxyalkyl, phenoxyalkoxy or phenoxyalkyl, cycloalkylalkyl substituted by C₆₋₂₀alkyl,
heteroarylalkyl substituted by C₆₋₂₀alkyl,
heterocyclic C₆₋₂₀alkyl or
heterocyclic alkyl substituted by C₂₋₂₀alkyl,
and wherein
the alkyl moiety may have in the carbon chain, a bond or a heteroatom selected from a double bond, a triple bond, O, S, sulfinyl, sulfonyl, or NR₆, wherein R₆ is as defined above, and as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and each of R₂, R₃, R₄ and R₅, independently, is H, C₁₋₄ alkyl or acyl ; or a pharmacologically acceptable salt, solvate or hydrate thereof;
- a compound a compound of formula IVa or IVb 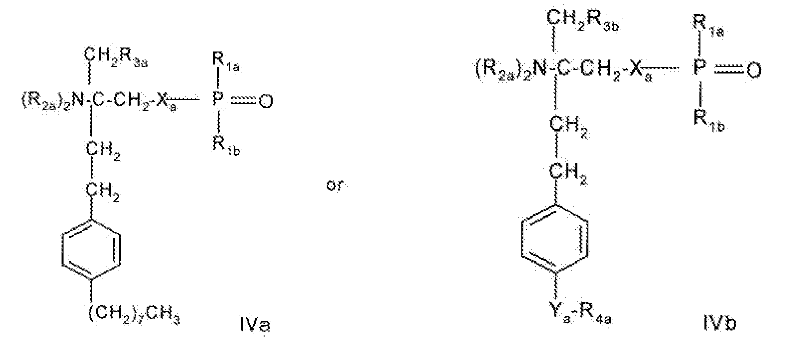 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂, is independently selected from H or (C₁₋₄)alkyl, which alkyl is unsubstituted or substituted by halogen; R₂ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-. -C(O)-, -CH(OH)-, -C(=NOH)-, O or S, and R₄ₐ is (C₄₋₁₄)alkyl or (C₄₋₁₄)alkenyl;
or a pharmacological acceptable salt, solvate or hydrate thereof;
- a compound of formula IX 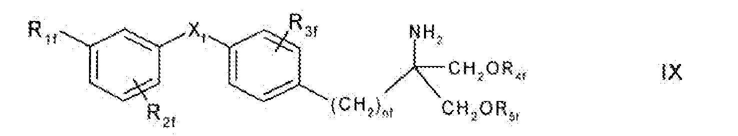 wherein X_{f} is O or S, and R_{1f}, R_{2f}, R_{3f} and n_{f} are as disclosed in WO 03/29184 and WO 03/29205. each of R_{4f} and R_{5f}, independently is H or a residue of formula 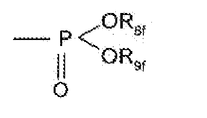 wherein each of R_{8f} and R_{9f}, independently, is H or C₁₋₄alkyl optionally substituted by halogen;
- a compound of formula XII 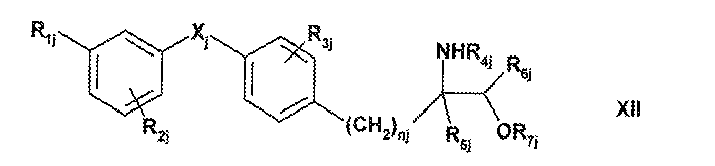 wherein
R₁ⱼ is halogen, trihalomethyl, C₁₋₄alkyl, C₁₋₄alkoxy, C₁₋₄alkylthio, C₁₋₄alkylsulifinyl, C₁₋₄alkylsulfonyl, aralkyl, optionally substituted phenoxy or aralkyloxy, R₂ⱼ is H, halogen, trihalomethyl, C₁₋₄alkyl, C₁₋₄alkoxy, aralkyl or aralkyloxy, R₃ⱼ is H, halogen, CF₃, C₁₋₄alkyl, C₁₋₄alkoxy, C₁₋₄alkylthio or benzyloxy, R_{Aj} is H, C₁₋₄alkyl, phenyl, optionally substituted benzyl or benzoyl, or lower aliphatic C ₁₋₅acyl, Rₛⱼ is H, monohalomethyl, C₁₋₄alkyl, C₁₋₄alkoxymethyl, C₁₋₄alkl-thiomethyl, hydroxyethyl, hydroxypropyl, phenyl aralkyl, C₂₋₄alkenyl or -alkynyl, each of R₆ⱼ and R₇ⱼ, independently, is H or C₁₋₄alkyl, or R₇ⱼ being also a residue of formula 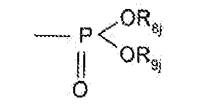 wherein each of R₈ⱼ and R₉ⱼ, independently, is H or C₁₋₄alkyloptionally substituted by halogen Xⱼ is O, S, SO or SO₂ and nⱼ is an integer of 1 to 4, e.g. 2-amino-4-[4-(3-benzyloxyphenylthio)-2-chlorophenyl]-2-methylbutane-1-ol or 2-amino-4-[4-(3-benzyloxyphenylthio)-2-chlorophenyl]-2-ethylbutane-1-ol;
- a compound of formula XIIIa or XIIIb 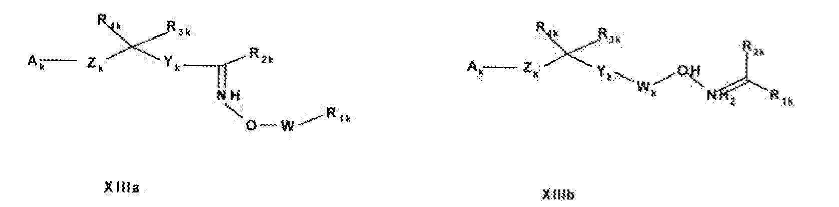 wherein
Aₖ is COOR₅ₖ, OPO(OR₅ₖ)₂, PO(OR₅ₖ)₂. SO₂OR₅ₖ, POR₅ₖOR₅ₖ or 1*H*-tetrazol-5-yl, R₅ₖ being H or C₁₋₆alkyl;
Wₖ is a bond, C₁₋₃alkyleneor C₂₋₃alkenylene;
Yₖ is C₆₋₁₀aryl or C₃₋₉heteroaryl, optionally substituted by 1 to 3 radicals selected from halogene, OH, NO₂, C₁₋₆alkyl, C₁₋₆alkoxy; halo-substituted C₁₋₆alkyl and halo-substituted C₁₋₆alkoxy;
Zₖ is a heterocyclic group as indicated in WO 04/103306A. e.g. azetidine;
R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cycloalkyl, C₃₋₈cycloalkylC₁₋₄alkyl, C₃₋₈heterocycloalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋₆alkyl or -C₁₋₆alkoxy;
R₂ₖ is H, C₁₋₆alkyl, halo substituted C₁₋₆alkyl, C₂₋₆alkenyl or C₂₋₆alkynyl; and each of R₃ₖ or R₄ₖ, independently, is H, halogen, OH, C₁₋₆alkyl, C₁₋₆alkoxy or halo substituted C₁₋₆alkyl or C₁₋₆alkoxy;
and the N-oxide derivatives thereof or prodrugs thereof,
or a pharmacologically acceptable salt, solvate or hydrate thereof.

2. Use according to claim 1 wherein the 31P receptor agonist is 2-amino-2-[2-(4-octylphenyl) ethyl]propane-1,3-diol, Compound B or C, (2R)-2-amino-4-[3-(4-cyclohexyloxybutyl)-benzo[b]thien-6-yl]-2-methylbutan-1-ol, or a compound of formula IX as defined hereinbefore wherein X_{f} is S or O, R_{1f} is benzyloxy, R_{2f}, R_{4f} and R_{5fj} are each H, R_{3f} is Cl and n_{f} is 2, in free form or in a pharmaceutically acceptable salt form.

3. Use according to according to claim 1 or claim 2, wherein the S1 P receptor agonist is 2-amino-2-12-(4-octylphenyl) ethyl]propane-1,3-diol (compound A) in free form or in a pharmaceutically acceptable salt form.

4. Use according to any preceding claim wherein said ocular disorder is selected from the group of ischemic retinopathies in general, anterior ischemic optic neuropathy, all forms of optic neuritis, age-related macular degeneration (AMD), in its dry forms (dry AMD) and wet forms (wet AMD), diabetic retinopathy, diabetic macular edema (DME), proliferative diabetic retinopathy (PDR), cystoid macular edema (CME), retinal detachment, retinitis pigmentosa (RP), Stargardt's disease, Best's vitelliform retinal degeneration, Leber's congenital amaurosis and other hereditary retinal degenerations, pathologic myopia, retinopathy of prematurity, and Leber's hereditary optic neuropathy, the after effects of corneal transplantation or of refractive corneal surgery, keratoconjunctivitis sicca (KCS) or dry eye and herpes keratitis.

5. Use according to according to any of the preceding claims, wherein said SIP receptor agonist is administered topically in or around the eye.

6. Use according to any preceding claims, wherein said ocular disorder is age-related macular degeneration (AMD), in its dry forms (dry AMD) and wet forms (wet AMD).
